# EUROPÄISCHE PATENTSCHRIFT

(11) **EP 3 622 921 B1**
(45) Veröffentlichungstag und Bekanntmachung des Hinweises auf die Patenterteilung: **04.08.2021**
(21) Anmeldenummer: 19178321.6
(22) Anmeldetag: 05.06.2019
(51) Int. Cl.: A61F 2/46

(54) **INSTRUMENT ZUM EINSETZEN UND EIN VERFAHREN ZUM FIXIEREN EINES GELENK-IMPLANTATS AUF EINEM INSTRUMENT KOPF**
INSTRUMENT FOR INSERTING AND A METHOD FOR FIXING AN ARTICULATED IMPLANT TO THE INSTRUMENT HEAD
INSTRUMENT DESTINÉ À ÊTRE INSÉRÉ ET PROCÉDÉ DE FIXATION D'UN IMPLANT D'ARTICULATION À LA TÊTE D'INSTRUMENT

(30) Priorität: 13.09.2018 DE 102018122344; 15.01.2019 DE 102019100868
(43) Veröffentlichungstag der Anmeldung: 18.03.2020
(73) Patentinhaber: Mathys AG Bettlach, 2544 Bettlach (CH)
(72) Erfinder: Kovacevic, Darko, 5032 Aarau Rohr (CH); Klockow, Andreas, 4410 Liestal (CH); Stutz, Michelle, 8184 Bachenbülach (CH); Eitel, Frank, 4101 Oberwil Bl (CH); Saladin, Stefan, 4539 Rumisberg (CH)
(74) Vertreter: Körfer, Thomas

(56) Entgegenhaltungen:
- EP-A1- 2 213 261
- EP-A1- 2 836 135
- WO-A2-2012/024288
- US-A1- 2008 154 261

## Beschreibung

Die Erfindung betrifft ein Instrument zum Einsetzen eines Gelenk-Implantats in einen menschlichen oder tierischen Knochen und ein Verfahren zum Fixieren eines Gelenk-Implantats auf einem Instrument.

Aufgrund von Krankheiten, Unfällen oder auch Verschleißerscheinungen durch Überbelastung, können Gelenke des menschlichen oder des tierischen Körpers beschädigt werden. Oft muss dann das beschädigte Gelenk ganz oder teilweise durch ein künstliches Gelenk-Implantat ersetzt werden. Beispielsweise ist es beim menschlichen Körper häufig erforderlich Kniegelenke, Schultergelenke oder Hüftgelenke durch entsprechende Gelenk-Implantate zu ersetzten. Insbesondere die Implantation künstlicher Hüftgelenke gehört heute zu den erfolgreichsten Standardeingriffen in der Chirurgie. Das Ziel des Gelenkersatzes besteht in der Schmerzausschaltung, Wiederherstellung der Funktion und Rekonstruktion der physiologischen Anatomie des Hüftgelenks. Aufgrund der demographischen Entwicklung und der zunehmenden Bedeutung des Sports auch in höheren Lebensabschnitten ist von einer steigenden Zahl derartiger Operationen auszugehen. Daher ist neben einer stetigen Verbesserung der Implantate selbst und der Verbesserung der Operationstechniken auch das Instrumentarium zum Einsetzen der Implantate ständig weiterzuentwickeln, um einen optimalen Operations- und Heilungsverlauf zu gewährleisten.

Zum Einsetzen der Implantate, insbesondere zum Einsetzen von Gelenkspfannen in einen hierfür vorbereiteten Knochen, gibt es verschiedenste Instrumente, welche ein Fixieren des Implantats auf dem Instrument und ein Einsetzen des Implantats in den menschlichen oder tierischen Knochen ermöglichen.

Beispielsweise ist aus dem Produktinformationsblatt der Mathys AG Bettlach mit dem Titel "RM Pressfit, Operationstechnik", verfügbar unter http://www.mathysmedical.com/Storages/User/Dokumente/Opera tionstechnik/Huefte/OP-Technik_RM_Pressfit_D_V03.pdf, eine Hüftgelenkspfanne, RM Pressfit Pfanne genannt, bekannt und ein entsprechendes Instrument zum Einbringen der Hüftgelenkspfanne in den menschlichen Knochen wird gezeigt. Das Instrument zum Einbringen der Hüftpfanne ist auch aus US2008/0154261A1 bekannt.

Gemäß dem zitierten Produktinformationsblatt wird ein Gelenk-Pfannenimplantat, die RM Pressfit Pfanne, mit einem sogenannten Setzinstrument fixiert und in den entsprechend vorbereiteten Knochen eingebracht. Die RM Pressfit Pfanne weist sechs Bohrungen, die auch Pfannen-Führungslöcher genannt werden, auf. Das Setzinstrument besteht aus einem Handgriff mit einem Greifkopf, der mehrere bewegliche Stifte aufweist, wobei in den Handgriff mit Greifkopf eine Stange mit einer sogenannten Schlagplatte eingeführt wird. Auf den Greifkopf wird dann ein Zentrier-Kopf aus Kunststoff aufgesetzt, um das Gelenk-Pfannenimplantat zentriert auf dem Greifkopf aufsetzten zu können. Schließlich wird das Gelenk-Pfannenimplantat auf die beweglichen Stifte des Greifkopfs und dem Zentrier-Kopf aufgesetzt, wobei die Stifte in die Pfannen-Führungslöcher hineingeführt sind und fixiert werden, indem durch eine Drehbewegung der Schlagplatte die beweglichen Stifte aufgespreizt werden.

Nachteilig an diesem Instrument zum Einsetzen des Gelenk-Implantats ist, dass für jede Größe von RM Pressfit Pfannen ein anderer Handgriff mit Greifkopf verwendet werden muss, da die Pfannen-Führungslöcher einer bestimmten RM Pressfit Pfannengröße auch nur auf einen bestimmten Greifkopf passen. Des Weiteren muss die Schlagplatte mittels der Finger gedreht werden und kann nicht mit einer Drehung der Hand gedreht werden. Eine Drehung mit der Hand ist leichter und zuverlässiger durchzuführen ohne eventuell abzurutschen. Ein weiterer Nachteil besteht darin, dass dieses Instrument zum Einsetzen des Gelenk-Implantats aus vielen kleinen Teilen zusammengesetzt ist und diese zur Reinigung und Sterilisation nicht auseinander genommen werden können.

Gemäß dem zitierten Dokument wird eine Fixierung des Gelenk-Implantats dadurch erreicht, dass durch das Drehen der Schlagplatte ein radiales Verschieben der beweglichen Stifte erfolgt, so dass die Stifte zueinander immer parallel ausgerichtet sind, und somit auch immer parallel zur Längsachse der Pfannen-Führungslöcher ausgerichtet sind. Dies führt zu keiner optimalen Fixierung des Gelenk-Implantats am Greifkopf, da keine Kraft in Richtung der Auflagefläche des Implantats erzeugt wird.

Der Erfindung liegt daher die Aufgabe zugrunde, ein Instrument zum Einsetzen eines Gelenk-Implantats in einen menschlichen oder tierischen Knochen und ein Verfahren zum Fixieren eines Gelenk-Implantats auf einem Instrument zu schaffen, ohne die aufgeführten Nachteile in Kauf nehmen zu müssen.

Die Aufgabe wird bezüglich des Instruments zum Einsetzen eines Gelenk-Implantats durch die Merkmale des Anspruchs 1 gelöst, bezüglich des Verfahrens zum Fixieren des Gelenk-Implantats durch die Merkmale des Anspruchs 17. Vorteilhafte Weiterbildungen sind Gegenstand der hierauf rückbezogenen Unteransprüche.

Ein erfindungsgemäßes Instrument zum Einsetzen eines Gelenk-Implantats besteht aus einem Instrumenten-Kopf der mindestens einen beweglichen Stift aufweist, wobei der bewegliche Stift kippbar ist.

Vorteilhafterweise bietet der mindestens eine bewegliche Stift die Möglichkeit, ein Gelenk-Implantat mit einer entsprechenden Ausnehmung auf den Instrumenten-Kopf aufzusetzen, wobei die Ausnehmung derart ausgeformt ist, dass sie den zumindest einen beweglichen Stift aufnehmen kann. Es ist denkbar, dass das Gelenk-Implantat auf einen Zentrier-Kopf aufgesetzt wird, der selbst wiederum auf den Instrumenten-Kopf aufgesetzt ist, wodurch ein Verrutschen des Implantats verhindert ist. Die Möglichkeit den Stift, der sich innerhalb der Ausnehmung des aufgesetzten Gelenk-Implantats befindet, kippen zu können, erlaubt die Ausübung einer Kraft auf eine Innenfläche der Ausnehmung, um das Gelenk-Implantat auf dem Instrumenten-Kopf zu fixieren.

Darüber hinaus weist der Instrumenten-Kopf bevorzugt eine Auflagefläche für das Gelenk-Implantat auf und ist über einen Schaft mit einem Griff verbindbar. Dabei ist die Längsachse des beweglichen Stifts in einem ungekippten Zustand parallel zur Längsachse des Schafts ausgerichtet. Vorteilhafterweise ist der Instrumenten-Kopf mit einer Auflagefläche versehen, die es erlaubt, das Gelenk-Implantat aufzusetzen. Der Instrumenten-Kopf ist mit einem Schaft verbunden. Bevorzugt erfolgt diese Verbindung mittels eines Patronenverschlusses, wobei vorteilhafterweise der Greifer auf Seite des Schaftes und der Greifring auf Seite des Instrumenten-Kopfes angeordnet ist. Alternativ erfolgt diese Verbindung mittels einer Schraube die ein Gewinde aufweist, welches in eine Ausnehmung des Instrumenten-Kopfs, die mit einem Gewinde versehen ist, geschraubt wird. Schließlich ist vorteilhafterweise die dem Instrumenten-Kopf abgewandte Seite des Schaftes mit einem Griff versehen oder verbunden, der es dem Operateur ermöglicht das Instrument zum Setzten eines Gelenk-Implantats in der Hand zu halten und das daran fixierte Implantat in den menschlichen oder tierischen Körper einzubringen.

Des Weiteren ist es vorteilhaft, dass die Längsachse des beweglichen Stifts im ungekippten Zustand parallel zur Längsachse des Schaftes ist, da die Längsachsen der Ausnehmungen des Gelenk-Implantats zum Einführen des beweglichen Stifts ebenfalls parallel zur Längsachse des Schaftes sind. Das Gelenk-Implantat weist zumindest eine solche Ausnehmung auf. Bevorzugt weist das Gelenk-Implantat 3 oder mehr, beispielsweise 6 Ausnehmungen auf. Somit ist ein Aufsetzten des Gelenk-Implantats auf der Auflagefläche des Instrumenten-Kopfs und ein gleichzeitiges Einführen des Stifts in die zumindest eine Ausnehmung des Gelenk-Implantats ohne zu verkanten möglich.

Bevorzugt ist der zumindest eine bewegliche Stift derart kippbar, dass seine Längsachse im gekippten Zustand nicht parallel zu der Längsachse des Schafts ist. Vorteilhafterweise wird durch das Kippen des zumindest einen beweglichen Stifts eine Kraft auf einer Innenfläche der Ausnehmung des Gelenk-Implantats erzeugt. Diese Kraft beinhaltet auch einen Kraftanteil, der in Richtung der Auflagefläche des Instrumenten-Kopfs gerichtet ist, und somit eine Fixierung des Gelenk-Implantats auf der Auflagefläche bewirkt.

In einer weiteren vorteilhaften vorzugsweisen Ausgestaltung weist der Instrumenten-Kopf in seiner Auflagefläche mindestens einen feststehenden Stift auf, dessen Längsachse parallel zu der Längsachse des Schafts ist.

Vorteilhafterweise weist der Instrumenten-Kopf in seiner Auflagefläche mindestens einen feststehenden Stift auf. Dieser feststehende Stift, dessen Längsachse parallel zu der Längsachse des Schafts ist und auch parallel zu der Längsachse der Ausnehmung des Gelenk-Implantats zum Einführen des feststehenden Stifts, kann in die Ausnehmung des Implantats eingeführt werden. Dieser feststehende Stift fixiert das Gelenk-Implantat, so dass es in der Horizontalebene der Auflagefläche des Instrumenten-Kopfs nicht verschiebbar ist. Somit kann durch das Kippen des zumindest einen beweglichen Stifts das Implantat nicht verrutschen und das Fixieren des Implantats auf der Auflagefläche des Instrumenten-Kopfs wird unterstützt. Bei zumindest einem vorhandenen Stift ist keine weitere Fixierung des Implantats in der Horizontalebene nötig. Es ist auch vorteilhaft, mehrere feststehende Stifte, bevorzugt 2 oder 4 Stifte vorzusehen, um das Implantat noch besser und zuverlässiger am Instrumenten-Kopf zu fixieren.

Weiterhin vorteilhafterweise ist der zumindest eine bewegliche Stift bevorzugt derart ausgeformt, dass er in zumindest eine Ausnehmung des Gelenk-Implantats, welches auf der Auflagefläche des Instrumenten-Kopfs aufgesetzt ist, eingreift. Zusätzlich oder alternativ ist das Gelenk-Implantat durch den gekippten Stift auf der Auflagefläche fixiert.

Vorteilhafterweise ist bzw. sind der bewegliche Stift und/oder der feststehende Stift zylindrisch geformt und die Geometrie des beweglichen Stifts und/oder des feststehenden Stifts ist derart gewählt, dass der Stift exakt in eine Ausnehmung des Gelenk-Implantats eingeführt werden kann. Es ist auch denkbar, dass ein Spalt zwischen dem Stift und der Ausnehmung in radialer und/oder axialer Richtung von mindestens 0,05mm vorhanden ist, bevorzugt von 0,1mm bis 0,2mm. Es ist auch denkbar, dass alle vorhandenen Stifte, sowohl bewegliche als auch unbewegliche Stifte, dieselbe Länge aufweisen oder aber unterschiedliche Längen aufweisen.

Vorteilhafterweise führt ein Kippen des zumindest einen beweglichen Stifts, der in eine Ausnehmung des Gelenk-Implantats eingeführt ist, zu einer Fixierung des Implantats auf der Auflagefläche des Instrumenten-Kopfs. Die Kraft, die vom gekippten Stift auf die Innenfläche der Ausnehmung wirkt, kann das Implantat nicht entlang der Horizontalebene der Auflagefläche, also jener Ebene, welche senkrecht auf die Längsachse des Schafts und auch senkrecht auf die Längsachse der Ausnehmung steht, verschieben. Denn das Implantat ist entweder durch einen feststehenden Stift, durch einen Zentrier-Kopf oder durch andere Fixierungsmittel, wie beispielsweise einem Kragen, der entlang der Auflagefläche verläuft, fixiert. Ein Teil der vom gekippten Stift ausgeübten Kraft auf eine Innenfläche der Ausnehmung des Gelenk-Implantats, ist normal auf die Horizontalebene in Richtung der Auflagefläche gerichtet, und fixiert somit das Implantat auf der Auflagefläche.

Bevorzugt ist in den Instrumenten-Kopf eine Ausnehmung eingebracht, in welche eine bewegbare Wippe eingesetzt ist. Es ist denkbar, dass die bewegbare Wippe an einem seitlichen Rand in mittiger Position selbst eine Ausnehmung aufweist, um beispielsweise einen Bolzen zur Unterstützung einer Kippbewegung durch die Ausnehmung zu führen.

Gemäß einer weiteren vorteilhaften bevorzugten Ausgestaltung des Instrumenten-Kopfs ist der zumindest eine bewegliche Stift auf der Wippe fixiert.

Es ist auch denkbar, dass auf der Wippe mehrere bewegliche Stifte fixiert sind.

Vorteilhafterweise wird auf der bewegbaren Wippe ein Stift fixiert, wodurch der Stift zum beweglichen Stift wird. Mittels einer entsprechenden Ausgestaltung der Wippe kann der darauf fixierte Stift derart bewegt werden, dass er entweder parallel zur Längsachse des Schafts ist, oder, dass er nicht parallel zur Längsachse des Schafts ist.

Bevorzugt wird die Wippe durch eine axiale Kraft entlang der Längsachse des Schafts derart bewegt, dass ihre der axialen Kraft zugewandte Fläche parallel zu einer Fläche ist, welche die axiale Kraft ausübt.

Vorteilhafterweise wird eine Bewegung der Wippe dadurch erreicht, dass eine Fläche in axialer Richtung, bezogen auf die Längsachse des Schafts, auf die Fläche der Wippe, welche der axial bewegbaren Fläche zugewandt ist, zubewegt wird. Die axial bewegte Fläche steht normal auf die Längsachse des Schafts und ist lediglich in axialer Richtung bewegbar. Somit erzwingt die sich axial auf die Wippe zubewegende Fläche eine Bewegung der Wippe derart, dass sich die Fläche der Wippe, welche der axial bewegbaren Fläche zugewandt ist, parallel zu dieser ausrichtet.

Bevorzugt ist die Fläche der Wippe, auf welche die axiale Kraft wirkt, nicht parallel zu der Fläche, auf welche der zumindest eine bewegliche Stift fixiert ist. Zusätzlich oder alternativ ist die Fläche, auf welche der zumindest eine bewegliche Stift fixiert ist, bevorzugt im ungekippten Zustand des beweglichen Stifts, parallel zur Auflagefläche des Instrumenten-Kopfs.

Vorteilhafterweise ist die der axial beweglichen Fläche zugewandte Fläche der Wippe nicht parallel zu der Fläche der Wippe, auf welcher der zumindest eine Stift fixiert ist. Somit führt eine axiale Kraft auf die Wippe zu einer Bewegung des Stifts, wobei die Bewegung einer Kippbewegung entspricht. Da die Fläche, auf welcher der zumindest eine bewegliche Stift fixiert ist, im ungekippten Zustand parallel zur Auflagefläche des Instrumenten-Kopfs ist, und somit normal zu der Längsachse des Schafts und auch normal zur Längsachse der Ausnehmung des Gelenk-Implantats zur Einführung des zumindest einen beweglichen Stifts ist, kann das Implantat auf den zumindest einen beweglichen Stift aufgesetzt werden. Dies ist einfach möglich, da die Seitenflächen des beweglichen Stifts parallel zu den inneren Seitenflächen der Ausnehmung im Gelenk-Implantat sind.

Ebenso bevorzugt wird die Wippe durch eine axiale Kraft entlang der Längsachse des Schafts über einen Bolzen im Instrumenten-Kopf bewegt. Dieser Bolzen seinserseits ist entlang seiner Längsachse im Instrumenten-Kopf beweglich und mit der Wippe gekoppelt.

Vorteilhafterweise ist die Längsachse des auf der Wippe fixierten Stiftes parallel zur Längsachse des Instrumenten-Kopfes solange dieser nicht mit dem Schaft verbunden ist. Das ist der ungekippte Zustand der Wippe. Die axiale Kraft entlang der Längsachse des Schafts wird durch Verbinden von Schaft und Instrumenten-Kopf erzielt. Die Verbindung Schaft - Instrumenten-Kopf geschieht über den entlang der Längsachse des Instrumenten-Kopfes beweglichen Bolzen. Die Bewegung der Wippe aus dem ungekippten in den gekippten Zustand wird dadurch erreicht, dass das dem Stift entgegengesetzte Ende der Wippe mit dem Bolzen gekoppelt ist.

Bevorzugt ist im entlang seiner Längsachse beweglichen Bolzen mindestens eine umlaufende Nut angebracht. In diese Nut greift das dem Stift entgegengesetzte Ende der Wippe ein. Damit wird eine Kupplung der Wippe mit dem beweglichen Bolzen erreicht. Das Verbinden von Schaft und Instrumenten-Kopf übt eine axiale Kraft entlang der Längsachse des Schafts, die dann mit der Längsachse des Instrumenten-Kopfes zusammenfällt, aus. Durch diese axiale Kraft wird die Wippe aus dem ungekippten in den gekippten Zustand gebracht. Der mindestens eine bewegliche Stift ist im gekippten Zustand nicht parallel zur Längsachse und fixiert so das Gelenk-Implantat. Diese Ausgestaltung bedingt, dass zuerst das Gelenk-Implantat auf den Instrumenten-Kopf gesetzt wird und erst dann der Instrumenten-Kopf mit aufgesetztem Gelenk-Implantat mit dem Schaft verbunden wird.

Bevorzugt wird die axiale Kraft erzeugt, indem das dem Griff abgewandte Ende des Schafts in die mit einem Greifring eines Patronenverschlusses versehene Ausnehmung des Instrumenten-Kopfs hineingedrückt wird und die am Schaft angebrachten Greifer des Patronenverschlusses einrasten. Damit ist der Patronenverschluss geschlossen und der Instrumenten-Kopf stabil mit dem Schaft verbunden.

Ebenso bevorzugt wird die axiale Kraft erzeugt, indem ein Gewinde, das am Schaft vorhanden ist und dessen Längsachse mit der Längsachse des Schafts zusammenfällt, in ein Gewinde des Instrumenten-Kopfs hineingedreht wird.

Vorteilhafterweise wird der Schaft durch das Einrasten des Patronenverschlusses oder alternativ durch das Hineindrehen des Schaftgewindes in das Gewinde des Instrumenten-Kopfs in Richtung des Instrumenten-Kopfs entlang der Achse des Schafts bewegt. Somit nähert sich auch die dem Instrumenten-Kopf zugewandte Fläche des Schafts dem Instrumenten-Kopf. Mit dieser Annäherung wird der Schaft in Richtung des Instrumenten-Kopfs gezogen und die unbewegliche Fläche des Schaftendes, die sich axial auf die Fläche der bewegbaren Wippe zubewegt, drückt schließlich gegen die Fläche der bewegbaren Wippe und erzeugt eine Kippbewegung der bewegbaren Wippe, bis die dem Schaft zugewandte Fläche der Wippe parallel zu der Fläche ist, die sich axial auf die Wippe zubewegt.

Alternativ wird der entlang der Längsachse bewegliche Bolzen durch das Einrasten des Patronenverschlusses oder alternativ durch das Hineindrehen des Schaftgewindes in das Gewinde des Instrumenten-Kopfs entlang der Längsachse des Schafts, die dann mit der Längsachse des Instrumenten-Kopfes zusammenfällt, bewegt. Die über die zumindest eine Nut des Bolzens gekoppelte, bewegbare Wippe wird aus dem ungekippten in den gekippten Zustand gebracht, womit auch der zumindest eine bewegliche Stift gekippt ist und das Gelenk-Implantat auf der Auflagefläche des Instrumenten-Kopfes fixiert ist.

Bevorzugt ist in den Instrumenten-Kopf eine Ausnehmung eingebracht, in welche das dem Griff abgewandte Ende des Schafts einführbar ist.

Vorteilhafterweise wird bei der Ausführungsvariante der Verbindung Schaft - Instrumenten-Kopf mittels Patronenverschluss das dem Griff abgewandte Ende des Schafts, welches den Patronenverschluss aufweist, in die Ausnehmung des Instrumenten-Kopfs eingeführt und damit der Patronenverschluss eingerastet.

Vorteilhafterweise wird bei der Ausführungsvariante der Verbindung Schaft - Instrumenten-Kopf mittels Schraube das dem Griff abgewandte Ende des Schafts, welches das Gewinde aufweist, in die Ausnehmung des Instrumenten-Kopfs eingeführt. Es ist denkbar, dass das dem Griff abgewandte Ende einen Sprengring oder eine Blattfeder enthält, so dass das Ende des Schafts in die Ausnehmung des Instrumenten-Kopfs hineingedrückt werden kann und einrastet.

Vorzugsweise wird auch ein Verfahren zum Fixieren eines Gelenk-Implantats mittels eines Instruments mit einem Instrumenten-Kopf, der mindestens einen beweglichen Stift aufweist, beschrieben. Das Verfahren beinhaltet insbesondere den Verfahrensschritt, dass das Gelenk-Implantat mittels zumindest einer Ausnehmung zum Aufnehmen eines beweglichen Stifts auf den beweglichen Stift des Instrumentenkopfs aufgesetzt wird. In einem weiteren Verfahrensschritt wird der vorhergenannte bewegliche Stift gekippt, um das Gelenk-Implantat auf dem Instrumentenkopf zu fixieren.

Vorteilhafterweise erlaubt das Verfahren zum Fixieren eines Gelenk-Implantats ein einfaches Fixieren eines Implantats, indem ein beweglicher Stift, der in eine Ausnehmung des Implantats eingeführt ist, gekippt wird.

Nachfolgend werden Ausführungsbeispiele der Erfindung anhand der Zeichnung beispielhaft beschrieben. In der Zeichnung zeigen:
- Fig. 1: ein erstes Ausführungsbeispiel des erfindungsgemäßen Instrumenten-Kopfs in Draufsicht, wobei die Stifte in die Zeichenebene hineinzeigen;
- Fig. 2: eine Schnittdarstellung des erfindungsgemäßen Instrumenten-Kopfs gemäß Fig. 1, wobei der Schnitt entlang der Schnittebene B-B verläuft;
- Fig. 3A: eine Schnittdarstellung des ersten Ausführungsbeispiels des erfindungsgemäßen Instrumenten-Kopfs mit einem beweglichen Stift, wobei die Schnittebene durch den beweglichen Stift im ungekippten Zustand verläuft;
- Fig. 3B: eine Schnittdarstellung des erfindungsgemäßen Instrumenten-Kopfs, wobei die Schnittebene durch den beweglichen Stift im gekippten Zustand verläuft;
- Fig. 3C: eine Schnittdarstellung des zweiten Ausführungsbeispiels des erfindungsgemäßen Instrumenten-Kopfs mit zwei beweglichen Stiften, wobei die Schnittebene durch die beweglichen Stifte im ungekippten Zustand verläuft;
- Fig. 3D: eine Schnittdarstellung des erfindungsgemäßen Instrumenten-Kopfs, wobei die Schnittebene durch die beweglichen Stifte im gekippten Zustand verläuft;
- Fig. 4: eine Draufsicht des erfindungsgemäßen Instrumenten-Kopfs, wobei die Stifte aus der Zeichenebene herauszeigen;
- Fig. 5: eine Seitenansicht eines Schafts der mit dem erfindungsgemäßen Instrumenten-Kopf verbindbar ist;
- Fig. 6: eine Schnittdarstellung des Schafts gemäß Fig. 5, wobei der Schnitt entlang der Schnittebene A-A geführt ist;
- Fig. 7A: eine Seitenansicht eines alternativen Schafts, der mit dem erfindungsgemäßen Instrumenten-Kopf mittels Schraube verbindbar ist;
- Fig. 7B: eine Seitenansicht eines alternativen Schafts, der mit dem erfindungsgemäßen Instrumenten-Kopf mittels Patronenverschluss verbindbar ist;
- Fig. 8: eine Draufsicht des alternativen Schafts gemäß Fig. 7A;
- Fig. 9A: eine Schnittdarstellung des alternativen Schafts gemäß Fig. 7A, wobei der Schnitt entlang der Schnittebene A-A gemäß Fig. 8 geführt ist;
- Fig. 9B: eine Schnittdarstellung des alternativen Schafts gemäß Fig. 7B, wobei der Schnitt entlang der Schnittebene A-A gemäß Fig. 8 geführt ist;
- Fig. 10A: ein drittes Ausführungsbeispiel des erfindungsgemäßen Instrument-Kopfs ohne eingelegte Wippe;
- Fig. 10B: die Wippe, welche in den erfindungsgemäßen Instrumenten-Kopf gemäß Fig. 10A einlegbar ist;
- Fig. 10C: eine Schnittdarstellung der Wippe gemäß Fig. 10B, welche in eine Ausnehmung des Instrumenten-Kopfs eingelegt ist, im ungekippten Zustand;
- Fig. 10D: eine Schnittdarstellung der Wippe gemäß Fig. 10B, welche in eine Ausnehmung des Instrumenten-Kopfs eingelegt ist, im gekippten Zustand;
- Fig. 11A: ein weiteres Ausführungsbeispiel des erfindungsgemäßen Instrumenten-Kopfs in Draufsicht, wobei die Stifte in die Zeichenebene hineinzeigen;
- Fig. 11B: eine Draufsicht des erfindungsgemäßen Instrumenten-Kopfs gemäß Fig. 11A, wobei die Stifte aus der Zeichenebene herauszeigen;
- Fig. 11C: eine Schnittdarstellung des erfindungsgemäßen Instrumenten-Kopfs gemäß Fig. 11A, wobei der Schnitt entlang der Schnittebene B-B verläuft;
- Fig. 12: eine Schnittdarstellung des alternativen Schafts analog Fig. 9B, wobei der Instrumenten-Kopf mit dem Schaft verbunden ist;
- Fig. 13: ein Ausführungsbeispiel eines Flussdiagramms für das erfindungsgemäße Verfahren zur Fixierung eines Gelenk-Implantats.

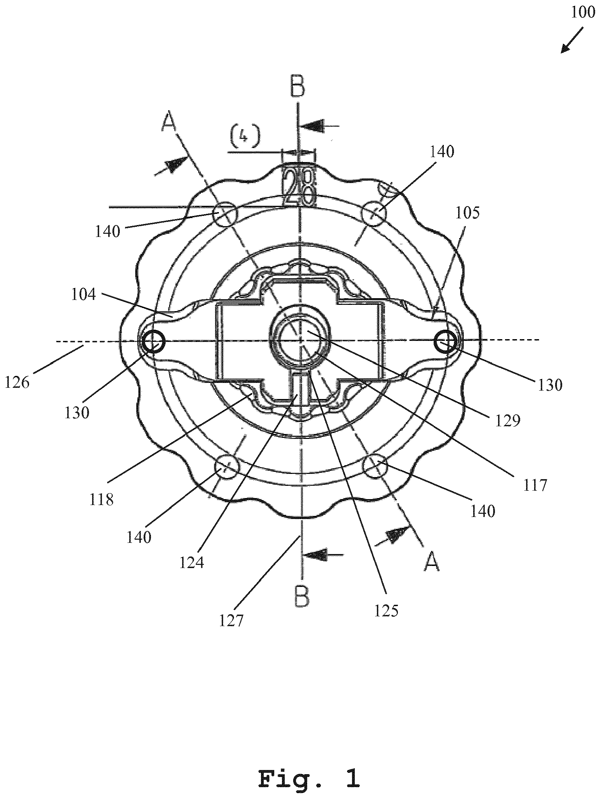

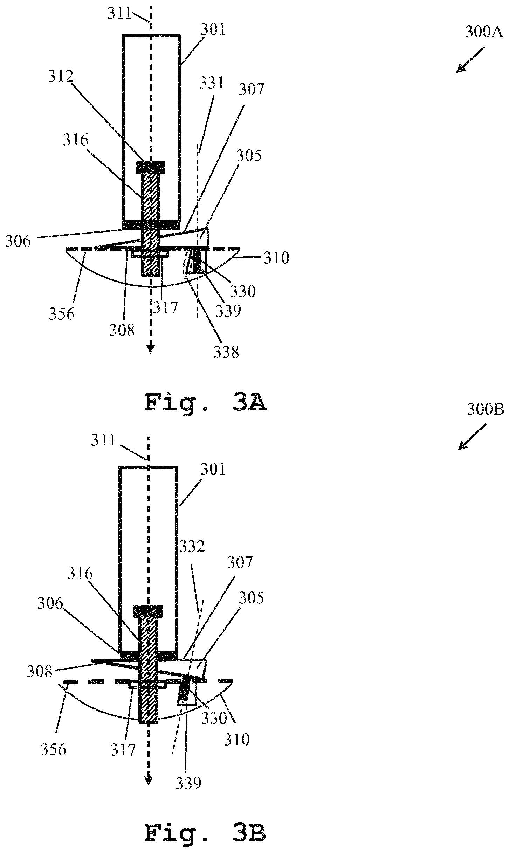

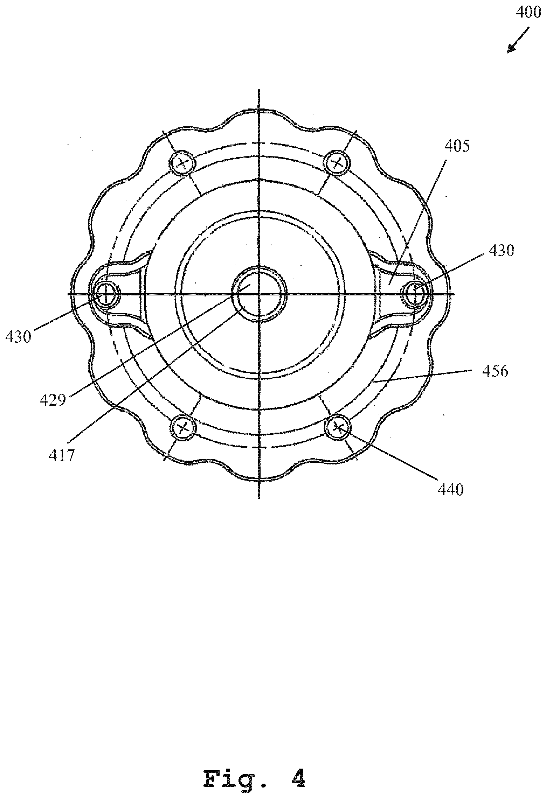

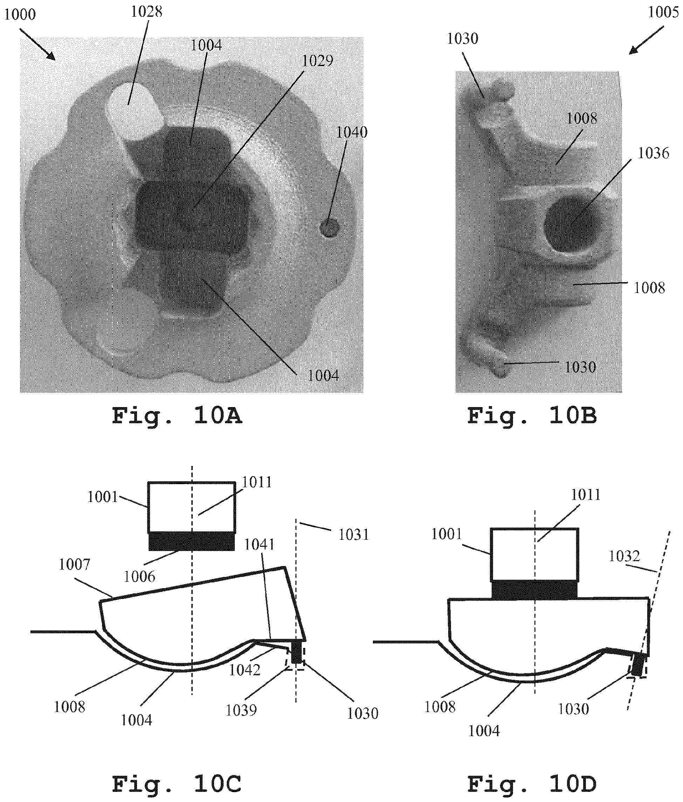

Die Bezugszeichen in Fig. 1 - 9 sind alle dreistellig gewählt und beginnen jeweils mit der der Figurennummer entsprechenden Zahl. Die Bezugszeichen in Fig. 10A, 10B, 10C und 10D sind vierstellig gewählt und beginnen mit der Zahl 10. Die Zehner- und Einer-Stellen der Referenznummern sind für identische Elemente in verschiedenen Figuren gleich gewählt. Beispielsweise wird der zumindest eine bewegliche Stift in Fig. 3A und Fig. 3B mit dem Bezugszeichen 330 bezeichnet, während der bewegliche Stift in Fig. 4 mit dem Bezugszeichen 430 und beispielsweise in Fig. 10B mit dem Bezugszeichen 1030 bezeichnet wird. Die Vorgehensweise wird analog für alle Figuren angewandt. Gleiche Elemente werden in einer Abbildung meist nur einmal beschriftet, um die Lesbarkeit der Abbildungen nicht zu beeinträchtigen.

Fig. 1 zeigt ein erstes Ausführungsbeispiel des erfindungsgemäßen Instrumentenkopfs.

Fig. 1 zeigt eine Draufsicht des Instrumenten-Kopfs 100 mit 4 feststehenden Stiften 140, welche auf dem Umfang eines Kreises verteilt sind. In den Instrumenten-Kopf 100 ist eine Ausnehmung 104 eingebracht, in welche eine Wippe 105 bewegbar eingelegt ist. Auf der Wippe, entlang ihrer Längsachse, sind zwei Stifte 130 fixiert, welche aufgrund der bewegbaren Wippe 105 bewegliche Stifte 130 sind. Die Wippe 105 ist eine in etwa rechteckige geometrische Form, welche entlang einer Drehachse 127, die normal zur Längsachse 126 der geometrischen Form steht, kippbar ist. Zur Unterstützung der Kippbewegung ist eine Ausnehmung 125 entlang der Drehachse 127 in die Wippe eingebracht, in welche ein Bolzen 124 eingreift. Alle Stifte gemäß der Fig. 1 zeigen in die Zeichenebene.

Die Wippe 105 weist im Schnittpunkt der Längsachse 126 und der Drehachse 127 eine Ausnehmung, bevorzugt eine Bohrung, auf, welche die Durchführung einer Schraube bzw. eines Gewindes erlaubt. Diese Ausnehmung der Wippe 105 ist annähernd deckungsgleich mit einer Ausnehmung 129 des Instrumenten-Kopfs 100. Die Ausnehmung 129 des Instrumenten-Kopfs 100 weist ein Gewinde 117, in welches eine Schraube bzw. ein Gewinde einschraubbar ist, auf.

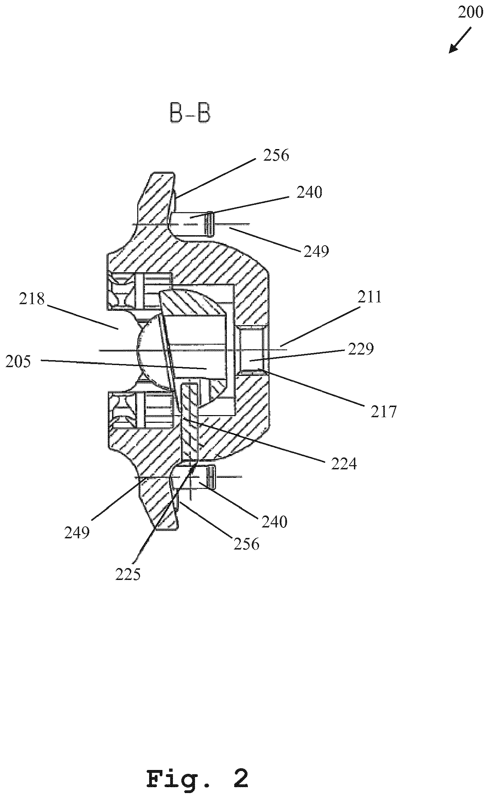

Zur Einführung eines Endes eines Schafts, ist in dem Instrumenten-Kopf eine weitere Ausnehmung 118 eingebracht. Ein Schaft, der an einem Ende einen Griff aufweist und an seinem anderen Ende ein Gewinde aufweist, ist mit jener Seite, die das Gewinde aufweist, in den Instrumenten-Kopf 100 einführbar. Das Gewinde des Schafts ist durch die Ausnehmung der Wippe 105, welche kein Gewinde aufweist durchführbar und in die Ausnehmung 129 im Instrumenten-Kopf 100, welche ein Gewinde 117 aufweist, einschraubbar. Fig. 2 zeigt einen Schnitt durch den erfindungsgemäßen Instrumenten-Kopf gemäß Fig. 1, wobei der Schnitt durch die Schnittebene B-B verläuft.

In Fig. 2 sind zwei feststehende Stifte 240 gezeigt, die je eine Längsachse 249 aufweisen. Die Längsachsen 249 der feststehenden Stifte 240 sind parallel zu einer Längsachse eines Schafts (nicht gezeigt), der mit dem Instrumenten-Kopf verbindbar ist.

Fig. 2 zeigt auch den Querschnitt einer Auflagefläche 256, auf welcher sich die feststehenden Stifte 240 befinden, und auf welche ein Gelenk-Implantat aufsetzbar ist.

In den Instrumenten-Kopf ist die Wippe 205 eingelegt, welche eine Ausnehmung 225 aufweist, in welche ein Bolzen 224 eingreift. Hierdurch wird eine Kippbewegung der Wippe 205 unterstützt bzw. geführt.

Fig. 3A zeigt eine Schnittdarstellung des ersten Ausführungsbeispiels des erfindungsgemäßen Instrumenten-Kopfs mit einem beweglichen Stift, wobei die Schnittebene durch den beweglichen Stift im ungekippten Zustand verläuft.

Fig. 3A zeigt die bewegbare Wippe 305, welche in eine Ausnehmung (nicht gezeigt) im Instrumenten-Kopf 300A eingesetzt ist. Auf der bewegbaren Wippe ist ein Stift 330 fixiert. Da dieser Stift 330 auf der bewegbaren Wippe fixiert ist, wird dieser konsequenterweise als beweglicher Stift 330 bezeichnet. In Fig. 3A ist die Auflagefläche 356 zu sehen (gestrichelt dargestellt), welche einen Teil des Instrumenten-Kopfs 300A bildet, und auf welcher beispielsweise feststehende Stifte (nicht gezeigt) fixierbar sind, oder auf welcher alternativ oder zusätzlich ein Kragen zur Fixierung eines Gelenk-Implantats in der Normalebene zur Längsachse 311 des Schafts 301 angebracht ist. Die Auflagefläche 356 ist nicht bewegbar und steht immer normal auf die Längsachse 311 des Schafts 301, der mit dem Instrumenten-Kopf 300A verbindbar ist.

Der Schaft 301 weist ein Gewinde 316, bevorzugt ein Außengewinde, auf, welches aus einer dem Instrumenten-Kopf 300A zugewandten Endfläche 306 austritt. Ein erstes Ende des Gewindes 316 weist beispielsweise einen Kopf 312, bevorzugt einen Zylinderkopf mit einer Innensechskantschraube, auf, um das Gewinde drehen zu können. Diese Drehung kann durch einen Griff mit einem Stab, der an dem dem Griff abgewandten Ende einen geeigneten Sechskantschlüssel aufweist, erreicht werden. Es ist aber auch denkbar, dass das Gewinde 316 im Schaft 301 unbeweglich fixiert ist, und dass das Gewinde 316 durch Drehen des Schafts 301 drehbar ist. Ein zweites Ende des Gewindes 316 ist gemäß Fig. 3A durch eine Ausnehmung, bevorzug eine Bohrung, in der Wippe 305 geführt und in ein Gewinde 317 im Instrumenten-Kopf 300A einschraubbar.

Fig. 3A zeigt die Wippe 305 in einem ungekippten Zustand. Die der Auflagefläche 356 zugewandte Fläche 308 der Wippe 305 ist parallel zur Auflagefläche 356, und ist somit normal zur Längsachse 311 des Schafts 301 orientiert. Die Längsachse 331 des beweglichen Stifts 330 ist parallel zu der Längsachse 311 des Schafts 301 angeordnet. Beim Aufsetzten eines Gelenk-Implantats 310 auf die Auflagefläche 356 des Instrumenten-Kopfs kann der bewegliche Stift 330 in eine entsprechende Ausnehmung 339 des Gelenk-Implantats 310 eingeführt werden, ohne zu verklemmen. Die Ausnehmung 339 des Gelenk-Implantats ist bevorzugt derart ausgeformt, dass zumindest eine Hinterschneidung 338 auf der dem Gewinde zugewandten Seite vorhanden ist. Auf diese Seite hin ist auch der beweglich Stift 330 kippbar.

Fig. 3B zeigt nun eine Schnittdarstellung des erfindungsgemäßen Instrumenten-Kopfs, wobei die Schnittebene durch den beweglichen Stift im gekippten Zustand verläuft.

In Fig. 3B ist die dem Schaft 301 zugewandte Fläche 307 der Wippe 305 nun parallel zu der Fläche 306 des Schafts 301 ausgerichtet. Durch eine axiale Bewegung des Schafts 301 in Richtung der Wippe 305 wird eine axiale Kraft auf die Fläche 307 der bewegbaren Wippe 305 ausgeübt, wodurch die Fläche 307 derart bewegt wird, dass sich diese parallel zu der Fläche 306, welche die axiale Kraft ausübt, ausrichtet.

In Fig. 3B ist gezeigt, dass sich der bewegliche Stift 330 mit der bewegbaren Wippe bewegt und gekippt wird. Im gekippten Zustand ist die Längsachse 332 des Stifts 330 nicht mehr parallel zur Längsachse 311 des Schafts 301. Der Stift 330 ist auf jene Seite der Ausnehmung 339 des Gelenk-Implantats 310 gekippt, welche die Hinterschneidung 338 aufweist. Der gekippte Stift 330, der sich innerhalb der Ausnehmung 339 des Gelenk-Implantats 310 befindet, übt nun eine Kraft auf die Innenflächen der Ausnehmung 339 aus, nämlich auf die Fläche, welche die Hinterschneidung bildet, wobei eine Kraftkomponente normal zu und in Richtung der Auflagefläche 356 des Instrumenten-Kopfs 300B wirkt. Somit wird das Gelenk-Implantat durch den gekippten Stift 330 innerhalb der Ausnehmung 339 auf der Auflagefläche 356 fixiert. Bevorzugt, ist die Längsachse 332 des beweglichen Stifts 330 parallel zu der Fläche, welche die Hinterschneidung bildet und der Stift übt mit seiner gesamten, der Hinterschneidung zugewandten Fläche, eine Kraft auf die Fläche aus, welche die Hinterschneidung bildet. Dies erlaubt eine optimale Kraftausübung des gekippten Stifts 330 auf das Gelenk-Implantat 310 und somit eine optimale, zuverlässige Fixierung des Gelenk-Implantats 310 auf der Auflagefläche 356 des Instrumenten-Kopfs 300B.

Die axiale Kraft wird erzeugt, indem das Gewinde 316, welches aus der Fläche 306 des Schafts 301 hinausgeführt ist, in das Gewinde 317 des Instrumenten-Kopfs 300A, 300B hineingedreht wird. Bevorzugt werden Rechtsgewinde eingesetzt, so dass ein Rechtsdrehen des Gewindes 316 des Schafts 301 in das Gewinde 317 des Instrumenten-Kopfs 300A, 300B eine axiale Bewegung des Schafts 301 in Richtung der Wippe 305 bewirkt. Durch diese axiale Bewegung wird die Fläche 306 des Schafts 301 auf die dem Schaft zugewandte Fläche 307 zubewegt und erzeugt eine Kippbewegung der bewegbaren Wippe 305.

Die Fläche 306 des Schafts 301 ist nur in axialer Richtung bewegbar und steht ansonsten immer normal auf der Längsachse 311 des Schafts 301.

Es ist denkbar, dass die Drehbewegung des Gewindes 316 erzeugt wird, indem ein Kopf 312, der mit dem Gewinde verbunden ist, bevorzugt im Uhrzeigersinn mittels einem zum Kopf 312 passenden Werkzeug gedreht wird. Es ist auch denkbar, dass bei der Verwendung eines Linksgewindes eine Drehung gegen den Uhrzeigersinn erforderlich ist, um die axiale Bewegung in Richtung der Wippe 305 zu erzeugen. Verschiedenste Ausführungen des Kopfs 312 sind denkbar, um das Gewinde zu drehen. Bevorzugt wird eine Innensechskantschraube eingesetzt, um das Gewinde drehen zu können. Diese Drehung kann durch einen Griff mit einem Stab, der an dem, dem Griff abgewandten Ende einen geeigneten Sechskantschlüssel aufweist, erreicht werden.

Es ist auch denkbar, dass das Gewinde 316 fest mit dem Schaft 301 verbunden ist, wodurch ein Drehen des Schafts 301 die axiale Bewegung bewirkt.

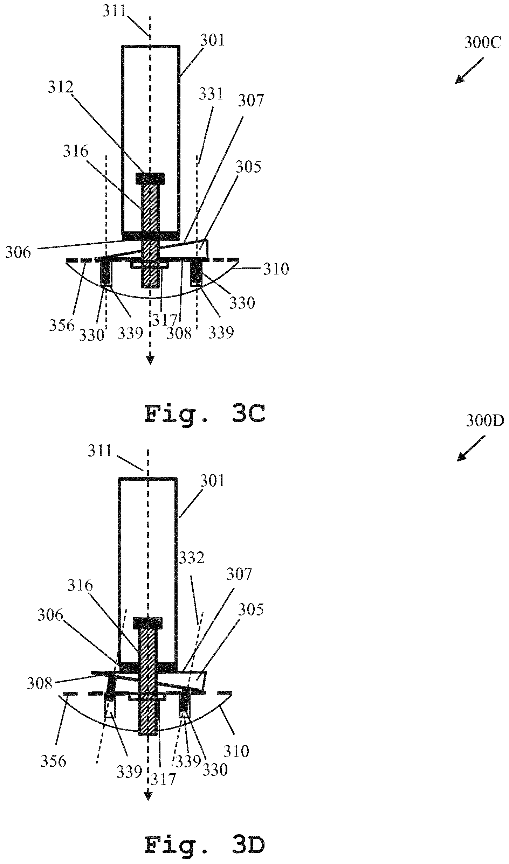

Fig. 3C zeigt eine Schnittdarstellung des zweiten Ausführungsbeispiels des erfindungsgemäßen Instrumenten-Kopfs mit zwei beweglichen Stiften, wobei der Schnitt durch die zwei beweglichen Stifte im ungekippten Zustand verläuft und das Gelenk-Implantat keine Ausnehmung mit Hinterschneidung aufweist. Es ist auch denkbar, ein Gelenk-Implantat mit zwei Ausnehmungen, welche eine Hinterschneidung aufweisen mit dem erfindungsgemäßen Instrumenten-Kopf zu fixieren.

Fig. 3C unterscheidet sich von der Darstellung in Fig. 3A lediglich durch die bewegbare Wippe 305, welche in eine Ausnehmung (nicht gezeigt) im Instrumenten-Kopf 300C eingesetzt ist und durch die Verwendung eines Gelenk-Implantats mit Ausnehmungen ohne Hinterschneidung. Auf der bewegbaren Wippe gemäß Fig. 3C sind nun zwei Stifte 330 fixiert. Da diese Stifte 330 auf der bewegbaren Wippe fixiert sind, werden diese konsequenterweise als bewegliche Stifte 330 bezeichnet.

Fig. 3C zeigt die Wippe 305 mit den zwei beweglichen Stiften 330 in einem ungekippten Zustand. Die der Auflagefläche 356 zugewandte Fläche 308 der Wippe 305 ist parallel zur Auflagefläche 356, und ist somit normal zur Längsachse 311 des Schafts 301 orientiert. Die Längsachsen 331 der beweglichen Stifte 330 sind parallel zu der Längsachse 311 des Schafts 301. Beim Aufsetzten eines Gelenk-Implantats 310 auf die Auflagefläche 356 des Instrumenten-Kopfs können somit die beweglichen Stifte 330 in entsprechende Ausnehmungen 339 des Gelenk-Implantats 310 eingeführt werden, ohne zu verklemmen.

Die Ausnehmungen weisen keine Hinterschneidung auf und erlauben ein annähernd passgenaues Einbringen der beweglichen Stifte 330 im ungekippten Zustand. Die Längsachsen der Ausnehmungen 339 verlaufen parallel zu der Längsachse 311 des Schafts 301. Es ist auch denkbar, ein Gelenk-Implantat mit einer ersten Ausnehmung 339 mit Hinterschneidung 338 und mit einer zweiten Ausnehmung 339 ohne Hinterschneidung mit dem erfindungsgemäßen Instrumenten-Kopf zu fixieren.

Fig. 3D zeigt nun eine Schnittdarstellung des erfindungsgemäßen Instrumenten-Kopfs, wobei der Schnitt durch die beweglichen Stifte im gekippten Zustand verläuft.

In Fig. 3D ist gezeigt, dass sich die zwei beweglichen Stifte 330 mit der bewegbaren Wippe bewegen und gekippt werden. Im gekippten Zustand ist die Längsachse 332 der Stifte 330 nicht mehr parallel zur Längsachse 311 des Schafts 301, und somit auch nicht mehr parallel zu der Längsachse der Ausnehmungen 339 im Gelenk-Implantat 310. Die gekippten Stifte 330 innerhalb der Ausnehmungen 339 des Gelenk-Implantats 310 üben eine Kraft auf die Innenflächen der Ausnehmungen 339 aus, wobei eine Kraftkomponente normal zu und in Richtung der Auflagefläche 356 des Instrumenten-Kopfs 300B wirkt. Somit wird das Gelenk-Implantat durch die gekippten Stifte 330 innerhalb der Ausnehmungen 339 auf der Auflagefläche 356 fixiert.

Die Erzeugung der axialen Kraft ist bereits in Bezug auf die Fig. 3B beschrieben und auch für Fig. 3D identisch. Fig. 4 zeigt eine Draufsicht des erfindungsgemäßen Instrumenten-Kopfs, wobei die Stifte aus der Zeichenebene herauszeigen.

Fig. 4 zeigt die Rückseite des Instrumenten-Kopf 100 aus Fig. 1. In der Darstellung ist die Anordnung sowohl der beweglichen Stifte 430 als auch der feststehenden Stifte 440 zu sehen. Fig. 4 zeigt auch eine Ausnehmung 429, bevorzugt eine Bohrung im Instrumenten-Kopf 400, die ein Gewinde 417, beispielsweise ein Innengewinde aufweist, in welches das Gewinde 316 einschraubbar ist. Die Stifte liegen alle entlang des Umfangs eines Kreises, dessen Mittelpunkt mit dem Mittelpunkt der Bohrung 429 zusammenfällt. Entlang dieses Kreisumfangs ist eine Auflagefläche 456 ausbildet, die es erlaubt, das Gelenk-Implantat 310 mit jener Seite, in welche ein Gelenkskopf einsetzbar ist, aufzusetzen. Im ungekippten Zustand sind die Längsachsen aller Stifte 330, 340 parallel zu der Längsachse 311 des Schafts 301 und auch zu den Längsachsen der Ausnehmungen 339. Somit ist es möglich, das Gelenk-Implantat 310 auf die Auflagefläche 456 aufzusetzen, wobei gleichzeitig die Stifte 330, 340 in die Ausnehmungen 339 des Gelenk-Implantats 310 eingeführt werden.

Es ist auch denkbar, dass ein Zentrier-Kopf auf den Instrumenten-Kopf 400 aufsetzbar ist, der bevorzugt auch Ausnehmungen, bevorzugt Bohrungen zum Durchführen der Stifte 330, 340, aufweist.

Ein Gelenk-Implantat 310 wird dann auf den Zentrier-Kopf aufgesetzt, wobei der Zentrier-Kopf eine Ausformung aufweist, welche passgenau in die Ausnehmung des Gelenk-Implantats 310, welche für den Gelenkskopf vorgesehen ist, einführbar ist. Somit wird das Gelenk-Implantat mittig am Instrumenten-Kopf 300A zentriert und eine horizontale Bewegung, dies entspricht einer Bewegung innerhalb der Ebene, welche durch die Auflagefläche 456 gebildet ist, verhindert. Denkbar sind auch andere Mittel, um ein Zentrieren des Implantats zu erreichen und ein Verrutschen zu verhindern, wie beispielsweise ein Kragen, der entlang des Kreisumfangs, der die Auflagefläche 456 umgibt, ausgeformt ist.

Da ein Gewinde 316 des Schafts 301 in den Instrumenten-Kopf 400 einschraubbar ist, kann ein und derselbe Schaft 301 mit einem dazugehörigen Griff dazu verwendet werden, verschiedene Instrumenten-Köpfe aufzuschrauben.

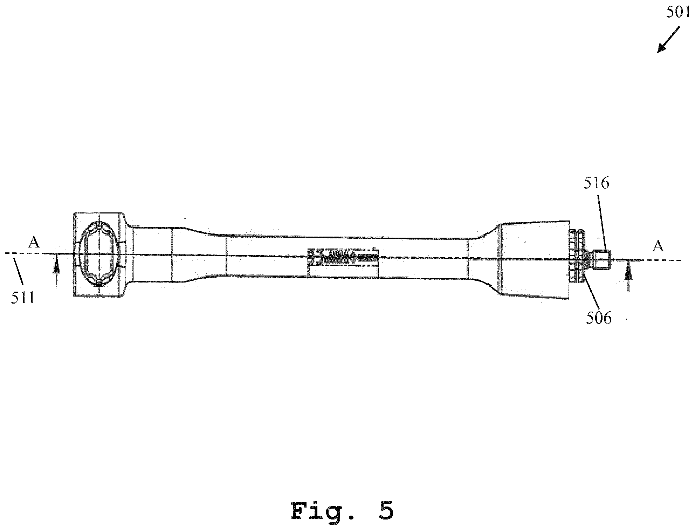

Fig. 5 zeigt eine Seitenansicht eines Schafts 501 der mit dem erfindungsgemäßen Instrumenten-Kopf 100 mittels Gewinde verbindbar ist. Zu sehen ist die Längsachse 511 des Schafts, welche mit der Längsachse des Gewindes 516 zusammenfällt. Die Fläche 506 des Schafts 501, aus welcher auch das Gewinde 516 herausgeführt ist, ist als Kreisfläche ausgeformt. Das Gewinde 516 kann beweglich ausgeführt sein, wodurch es mittels eines Werkzeugs gedreht werden kann, oder aber das Gewinde kann starr mit dem Schaft verbunden werden, womit das Gewinde nur durch Drehen des Schafts 501 gedreht werden kann.

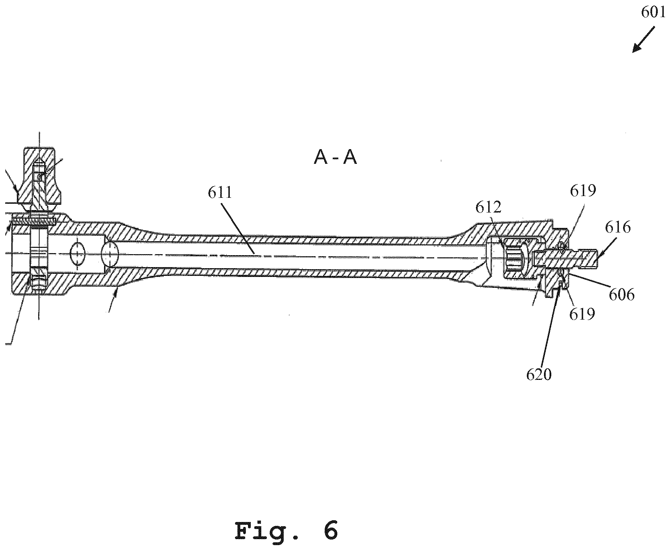

Der Schaft 501 wird in Fig. 6, welche einen Schnitt durch die Schnittebene A-A, welche mit der Längsachse 511 zusammenfällt, näher beschrieben. Fig. 6 zeigt einen Schnitt des Schafts gemäß Fig. 5, wobei der Schnitt entlang der Schnittebene A-A geführt ist.

3In Fig. 6 ist nun ein Teil des Innenlebens des Schafts 601 dargestellt. Das Gewinde 616 weist an einem ersten Ende einen Kopf 612 auf, bevorzugt einen Schraubenkopf mit beispielsweise einer Innensechskantschraube, in welche ein entsprechendes Gegenstück eingreifen kann, um das Gewinde 616 in Drehung zu versetzen. Das zweite Ende des Gewindes 616 wird, wie bereits in Fig. 3A und Fig. 3B gezeigt, durch eine Bohrung der Wippe 305 geführt und ist in das Gewinde 317 des Instrumenten-Kopfs 300A, 300B hineindrehbar. Zur Fixierung des Gewindes 616 im Schaft 601 sind zwei gegenüberliegende Klemmstifte 619 vorhanden. Diese erlauben eine Drehbewegung des Gewindes 616, verhindern aber eine axiale Bewegung des Gewindes und somit konsequenterweise auch ein Herausfallen des Gewindes.

Das mit dem Instrumenten-Kopf 300A, 300B verbindbare Ende des Schafts 601 weist beispielsweise eine ringförmige Nut auf, in welche ein Federelement 620, bevorzug ein Sprengring eingelegt wird. Wenn das Ende des Schafts 601 in eine Ausnehmung im Instrumenten-Kopf 300A, 300B eingeführt wird, welche derart ausgeformt ist, dass sich das Ende des Schafts in die Ausnehmung hineindrücke lässt, sodass das Federelement 620 in der Ausnehmung einrastet, lässt sich der Instrumenten-Kopf 300A, 300B einfach mit dem Schaft 601 verbinden. Die Fixierung des Schafts 601 und des Instrumenten-Kopfs 300A, 300B erfolgt dann, indem das Gewinde des Schafts 616 in das Gewinde 317 des Instrumenten-Kopfs 300A, 300B eingeschraubt wird.

Der Schaft weist an dem dem Gewinde abgewandten Ende eine Ausnehmung auf, die sich bis zum Kopf 612 erstreckt. Durch diese Ausnehmung ist ein Stab mit einem entsprechenden Gegenstück zum Kopf 612 einführbar, wobei das Gegenstück in den Kopf eingreift. Durch Drehen des Stabs (nicht dargestellt), dessen Ende in den Kopf 612 des Gewindes 616 eingreift, ist das Gewinde 616 drehbar. Um den Stab zunächst zu fixieren, bevor er zum Drehen des Gewindes 616 eingesetzt wird, ist eine Fixiervorrichtung vorhanden. Die Fixiervorrichtung erlaubt durch die Drehung eines Hebels die Fixierung des Stabs. Wenn ein Gelenk-Implantat 310 auf dem Instrumenten-Kopf 300A, 300B aufgesetzt ist und die Stifte des Instrument-Kopfs 330, 340 in die dafür vorgesehenen Ausnehmungen des Implantats eingeführt sind, wird die Fixiervorrichtung gelöst, sodass der Stab drehbar ist. Dann ist das Gewinde 616 des Schafts 601 in das Gewinde 317 (nicht gezeigt) des Instrumenten-Kopfs 300A, 300B hineindrehbar und die beweglichen Stifte 330 werden gekippt.

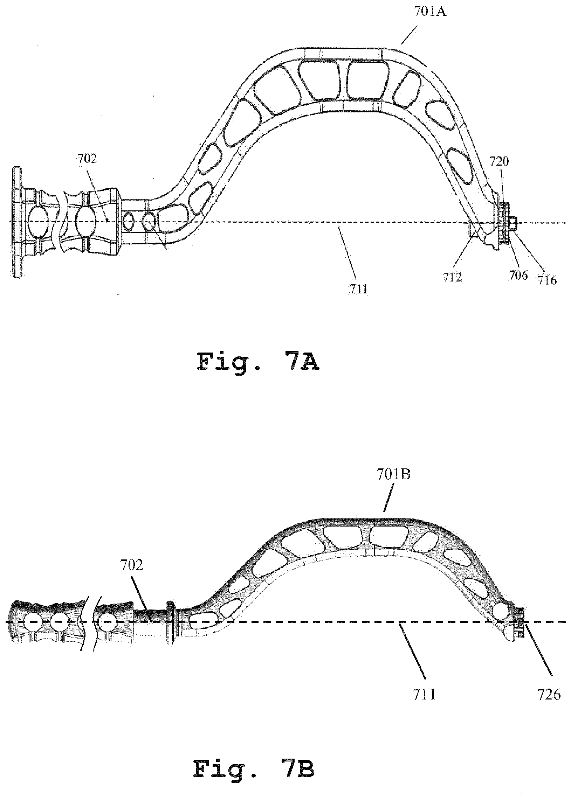

Fig. 7A zeigt eine Seitenansicht eines alternativen Schafts, der mit dem erfindungsgemäßen Instrumenten-Kopf verbindbar ist. Diese alternative Ausführungsform ist insbesondere dann vorteilhaft, wenn Körperteile des Patienten umgangen werden müssen.

Der alternative Schaft 701 ist mit einem Griff 702 versehen und weist einen Bogen auf. Die Längsachse des Griffs 702 fällt mit der Längsachse des Gewindes 716 des Schafts 701 zusammen. Der Kopf 712 an einem ersten Ende des Gewindes 716 ist gemäß Fig. 7A nicht innerhalb des Schafts 701 angeordnet, sondern außerhalb des Schafts und somit von außen zugänglich. Mit einem entsprechenden Werkzeug, welches in eine Ausnehmung des Kopfs 712 eingreift, kann das Gewinde 716 gedreht werden. Der Bogen, den der alternative Schaft 701 bildet, erlaubt vorteilhafterweise ein einfaches Drehen des am Instrumenten-Kopf fixierten Implantats beim Einsetzen in einen Knochen. Der Bogen hat einen Abstand von der Längsachse des Schafts, was einen Hebel erzeugt. Wenn eine Kraft zum Verdrehen des Implantats am Bogen angreift, wird diese aufgrund der Hebelwirkung verstärkt.

Alternativ kann das Verbinden des Schaftes 701 mit dem Instrumenten-Kopf 1100 mittels eines Patronenverschlusses geschehen. Auch so kann ein und derselbe Schaft 701, 501 mit einem dazugehörigen Griff dazu verwendet werden, verschiedene Instrumenten-Köpfe aufzuschrauben.

Fig. 7B zeigt eine Seitenansicht eines alternativen Schafts 701, der mit dem erfindungsgemäßen Instrumenten-Kopf 1100 mittels Patronenverschluss verbindbar ist. Zu sehen ist die Längsachse 711 des Schafts, welche mit der Längsachse des Patronenverschlusses 726 zusammenfällt. Die Greifer des Patronenverschlusses 726 sind bei dieser erfindungsgemäßen Ausführung auf Seite des Schaftes angebracht. Der Greifring des Patronenverschlusses befindet sich am Instrumenten-Kopf 1100. Die umgekehrte Anordnung, Greifring am Schaft und Greifer am Instrumenten-Kopf ist gleichwertig (nicht gezeigt).

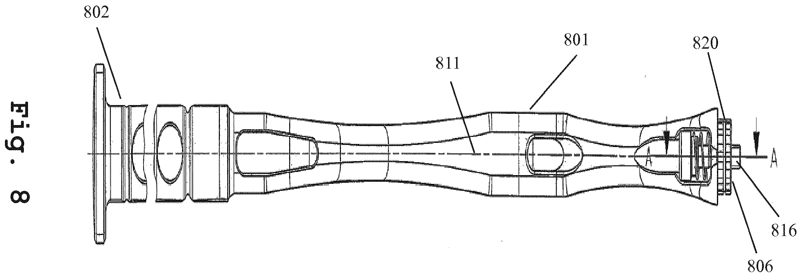

Fig. 8 zeigt eine Draufsicht des alternativen Schafts gemäß Fig. 7A. Fig. 8 zeigt insbesondere die Schnittebene A-A, welche mit der Längsachse 811 des Griffs 802 zusammenfällt.

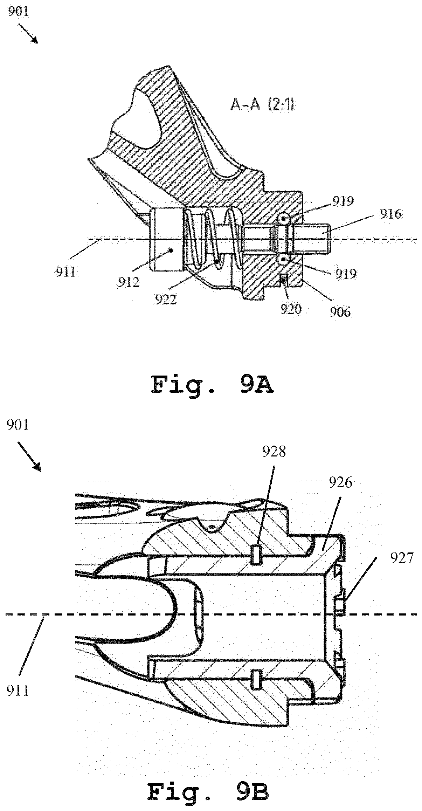

Fig. 9A zeigt einen Schnitt des alternativen Schafts mit Gewindeverbindung zum Instrumenten-Kopf entlang der Schnittachse A-A gemäß Fig. 8. In Fig. 9A ist das Gewinde 916, welches an einem ersten Ende einen Kopf 912 aufweist, gezeigt. Der Kopf des alternativen Schafts 901 befindet sich außerhalb des Schafts 901 und kann mit einem geeigneten Werkzeug, je nach Art des Kopfes, gedreht werden. Zu sehen sind in Fig. 9 auch im Detail die zwei Klemmstifte 919, welche das Gewinde 916 axial fixieren, aber eine Drehbewegung des Gewindes zulassen.

Da die Klemmstifte 919 eine Drehung des Gewindes 916 erlauben, und somit das Gewinde 916 nicht vollständig fixieren, ist eine axiale Hin-und-Her-Bewegung des Gewindes 916 möglich. Um ein Hin-und-Her-Bewegen des Gewindes 916 beim Einführen der Stifte 130, 140 und nach dem Lösen des Gewindes 916 zu vermeiden, ist eine Schraubendruckfeder 922 vorhanden.

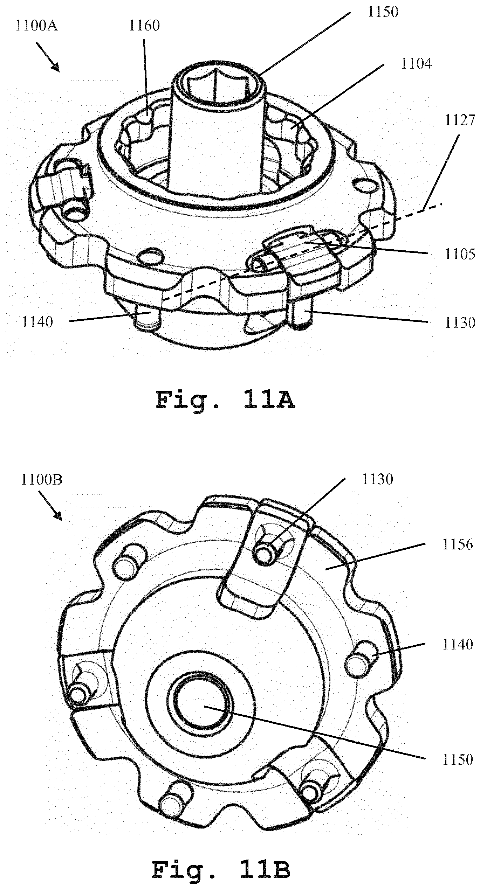

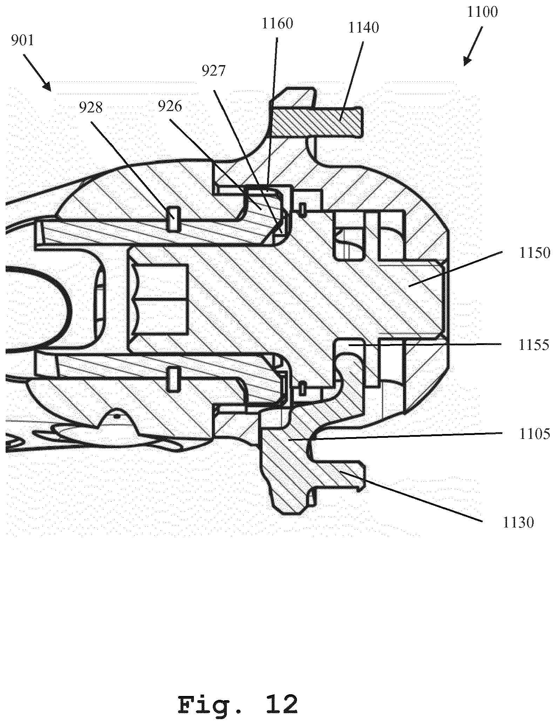

Fig. 9B zeigt einen Schnitt des alternativen Schafts mit Patronenverschlussverbindung zum Instrumenten-Kopf entlang der Schnittachse A-A gemäß Fig. 8. In Fig. 9B ist der hülsenförmige Greifer des Patronenverschlusses 926 gezeigt. Dieser ist über einen Sicherungsring 928 mit dem Rest des Schaftes verbunden. Die einzelnen Greifarme 927 rasten am als Zahnkranz 1160 (Fig. 11A) ausgebildeten Greifring des Instrumenten-Kopfes ein. Der Instrumenten-Kopf ist dann stabil mit dem Schaft verbunden, gezeigt in Fig. 12.

Fig. 10A zeigt ein drittes Ausführungsbeispiel des erfindungsgemäßen Instrumente-Kopfs 1000 ohne eingelegte Wippe 1005, welche in Fig. 10B im Detail gezeigt ist. Ohne die eingelegte Wippe 1005 sind die Ausnehmung 1004, in welche die Wippe einlegbar ist und auch die Ausnehmung 1029, in welcher ein Gewinde 1017 (nicht gezeigt) vorhanden ist, zu sehen. Die Ausnehmung 1004 ist nicht eben, sondern konkav ausgeformt, so dass eine konvex ausgeformte Fläche eingelegt werden kann. Des Weiteren sind auch zwei Ausnehmungen 1028 im Instrumenten-Kopf 1000 dargestellt, durch welche die Stifte 1030, welche auf der Wippe 1005 fixiert sind, durchführbar sind. Fig. 10A zeigt jene Seite des Instrumenten-Kopfs, welche beispielsweise mit dem Schaft 801 verbindbar ist. Wie bereits in vorhergehenden Ausführungen beschrieben, ist der Schaft 801 mit einem Gewinde 816 ausgeführt, welches in das Gewinde 1017 (nicht gezeigt) in der Ausnehmung 1029 des Instrumenten-Kopfs 1000, einschraubbar ist. In Fig. 10A ist auch ein feststehender Stift 1040 gezeigt, der mit dem Instrumenten-Kopf 1000 fest verbunden ist. Der feststehende Stift 1040 zeigt in die Bildebene hinein.

Fig. 10B zeigt nun die Wippe 1005, welche in den Instrumenten-Kopf 1000 einlegbar ist. Die Wippe 1005 weist zwei Stifte 1030 auf, welche auf einer Ebene liegen und symmetrisch zur Ausnehmung 1036 der Wippe 1005, durch welche das Gewinde 816 des Schafts 801 durchführbar ist, angeordnet sind. Aus Fig. 10B ist ersichtlich, dass in diesem Ausführungsbeispiel, im Gegensatz zu Fig. 1 und zu Fig. 4, die beiden Stifte 1030 nicht in einer Linie mit der Ausnehmung 1036 der Wippe 1005 liegen.

Die Fläche 1008, in der Nähe der Ausnehmung 1036 ist eine gekrümmte Fläche, bevorzugt eine konvexe Fläche, welche so ausgeformt ist, dass die Wippe 1005 mit seiner Fläche 1008 in die Ausnehmung 1004 des Instrumenten-Kopfs 1000 eingelegt werden kann und bewegbar ist. Wie durch Einwirken einer axialen Kraft auf eine Fläche der Wippe 1005 eine Kippbewegung der beweglichen Stifte 1030 erreicht wird, ist in Fig. 10C und 10D näher erläutert.

Fig. 10C zeigt eine Schnittdarstellung der Wippe gemäß Fig. 10B, welche in die Ausnehmung 1004 des Instrumenten-Kopfs 1000 eingelegt ist, wobei die Wippe 1005 sich im ungekippten Zustand befindet und mit seiner Fläche 1007 einem Schaft 1001 zugewandt ist. Die Wippe 1005 weist auf der dem Schaft 1001 abgewandten Seite eine konvexe Fläche 1008 auf, welche im Bereich des Stifts 1030 in eine ebene Fläche 1041 übergeht. Bevorzugt ist diese ebene Fläche 1041 im ungekippten Zustand parallel zu der Fläche 1006 des Schafts 1001, welche die axiale Kraft auf die Wippe 1005 ausübt. Die konvexe Fläche 1008 liegt beweglich in einer konkav ausgeformten Ausnehmung 1004 des Instrumenten-Kopfs 1000, so dass der Stift 1030 im ungekippten Zustand eine Längsachse 1031 aufweist, welche parallel zur Längsachse 1011 des Schafts 1001 ist. Die konkav ausgeformte Ausnehmung 1004 geht ebenfalls in eine ebene Fläche 1042 im Bereich des Stifts 1030 über, wobei diese ebene Fläche 1042 nicht parallel zu der ebenen Fläche 1041 der konvexen Fläche im Bereich des Stifts 1030 ist.

Gemäß Fig. 10C ist der Stift 1030 in eine Ausnehmung 1039 eingeführt. Diese Ausnehmung 1039 ist in einem Gelenk-Implantat eingebracht, welches auf dem Instrumenten-Kopf 1000 fixiert werden soll. Zur Erläuterung des erfindungsgemäßen Instrumenten-Kopfs ist in Fig. 10C lediglich die Ausnehmung 1039 dargestellt (strichliert) und nicht das vollständige Gelenk-Implantat.

Fig. 10D zeigt nun die Schnittdarstellung der Wippe 1005 gemäß Fig. 10B, welche in die Ausnehmung 1004 des Instrumenten-Kopfs 1000 eingelegt ist, im gekippten Zustand. Die dem Instrumenten-Kopf 1000 zugewandte Fläche 1006 des Schafts 1001 übt eine axiale Kraft auf die Fläche 1007 der Wippe aus, so dass die Fläche 1007 der Wippe parallel zu der Fläche 1006 des Schafts 1001 ausgerichtet wird. Aufgrund der konkav ausgeformten Ausnehmung 1004 des Instrumenten-Kopfs 1000 und der entsprechend konvex geformten Fläche 1008 der Wippe 1005, ist eine Kippbewegung der Wippe 1005 und somit eine Kippbewegung der beweglichen Stifte 1030 einfach und ohne hohen konstruktiven Aufwand zu erreichen. Die Ausformung der ebenen Fläche 1041 im Bereich des Stiftes 1030 und die entsprechende Ausformung der ebenen Fläche 1042 im Bereich des Stifts 1030, erlauben die Bestimmung einer Endposition des Stifts 1030 bei Parallelität der Fläche 1006 und der Fläche 1007. Dies wird gemäß Fig. 10D dadurch erreicht, dass bei der maximal gewünschten Kippbewegung der Wippe, 1005, die gesamte ebene Fläche 1041 auf der gesamten ebenen Fläche 1041 aufliegt, dass also die Fläche 1041 parallel zu der Fläche 1042 ist, und so die Wippe stabilisiert wird.

Die Neigung der ebenen Fläche 1041 ändert sich mit der Kippbewegung der Wippe 1005, die Neigung der ebenen Fläche 1042 wird durch die Konstruktion vorgegeben und änderst sich nicht durch eine Bewegung der Wippe. Diese Bestimmung der Endposition des Stifts 1030 verhindert ein zu starkes Kippen des Stiftes 1030 in Richtung der Ausnehmung 1039, was eine Beschädigung des Gelenk-Implantats hervorrufen könnte, und verhindert eine ungewollte Bewegung der Wippe 1005 bei maximal gekipptem Stift 1030 verhindert.

Es sind verschieden Ausgestaltungen der Wippe und der Ausnehmung innerhalb des Instrumenten-Kopfs zum Einlegen der Wippe denkbar, um eine gewünschte Kippbewegung der Wippe und somit der beweglichen Stifte zu erreichen. Die Verwendung mehrerer beweglicher Stifte als auch mehrerer feststehender Stifte in Kombination mit einem Gelenk-Implantat mit Ausnehmungen mit Hinterschneidungen oder ohne Hinterschneidungen ist denkbar.

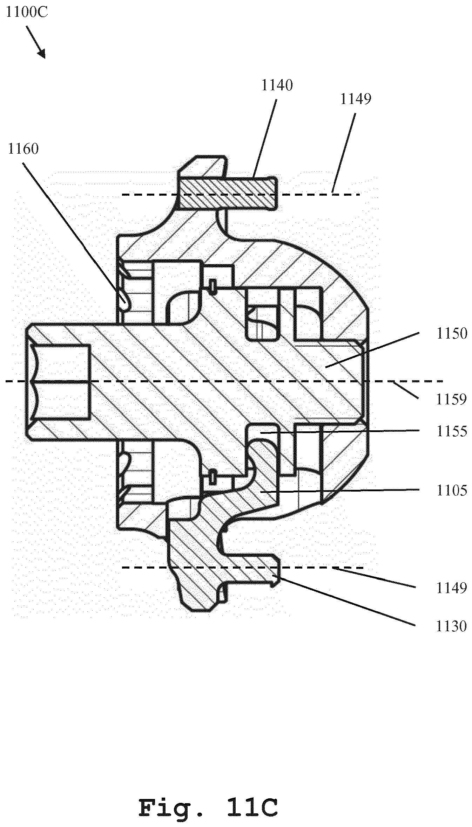

Fig. 11A, 11B und 11C zeigen ein weiteres Ausführungsbeispiel des erfindungsgemäßen Instrumentenkopfs.

Fig. 11A zeigt eine perspektivische Draufsicht des Instrumenten-Kopfs 1100 mit 3 feststehenden Stiften 1140, welche auf dem Umfang eines Kreises verteilt sind. In den Instrumenten-Kopf 1100 ist eine Ausnehmung 1104 eingebracht, in welche ein in seiner Längsachse beweglicher Bolzen 1150 eingelegt ist. Der Bolzen 1150 ist gekoppelt mit in Ausführungsbeispiel drei bewegbaren Wippen 1105. Auf den Wippen ist je am aussen liegenden Ende je ein beweglicher Stift 1130 fixiert, welcher auf Grund der bewegbaren Wippe ein beweglicher Stift ist. Die beweglichen Stifte 1130 sind auf demselben Umfangskreis angebracht wie die feststehenden Stifte 1140. Die Wippen 1105 sind kippbar um eine Drehachse 1127, die in einer Ebene senkrecht zur Längsachse des Instrumenten-Kopfs liegt. Der Innenrand der Ausnehmung 1104 ist als Zahnkranz ausgebildet. Diese Zähne 1160 bilden den Greifring des Patronenverschlusses und dienen gleichzeitig als Verdrehsicherung zwischen Schaft und Instrumentenkopf.

Fig. 11B zeigt perspektivisch die Unterseite des Instrumenten-Kopfs 1100 aus Fig. 11A. In der Darstellung ist der in seiner Längsachse beweglicher Bolzen 1150 zu sehen. Fig. 11B zeigt die Anordnung sowohl der beweglichen Stifte 1130 als auch der feststehenden Stifte 1140. Die Stifte liegen alle entlang des Umfangs eines Kreises, dessen Mittelpunkt mit dem Mittelpunkt des Bolzens 1150 zusammenfällt. Entlang dieses Kreisumfangs ist eine Auflagefläche 1156 ausbildet, die es erlaubt, das Gelenk-Implantat 310 mit jener Seite, in welche ein Gelenkskopf einsetzbar ist, aufzusetzen. Im ungekippten Zustand sind die Längsachsen aller Stifte 1130, 1140 parallel zu der Längsachse 711 des Schafts 701 und auch zu den Längsachsen der Ausnehmungen 339 des Gelenk-Implantats. Somit ist es möglich, das Gelenk-Implantat 310 auf die Auflagefläche 1156 aufzusetzen, wobei gleichzeitig die Stifte 1130, 1140 in die Ausnehmungen 339 des Gelenk-Implantats 310 eingeführt werden.

In Fig. 11C ist ein feststehender Stift 1140 und ein beweglicher Stift 1130 gezeigt, die je eine Längsachse 1149 aufweisen. Die Längsachsen 1149 des feststehenden Stiftes 1140 wie auch des beweglichen Stiftes 1130 sind parallel zu einer Längsachse eines Schafts (nicht gezeigt), der mit dem Instrumenten-Kopf verbindbar ist. Die Längsachse des Schaftes fällt zusammen mit der Längsachse des Instrumenten-Kopfs, die wiederum mit der Längsachse 1159 des in dieser Achse beweglichen Bolzens 1150 zusammenfällt. Die Verbindung Schaft - Instrumenten-Kopf geschieht mittels Patronenverschluss. Die Zähne 1160 des Greifringes des Patronenverschlusses sind sichtbar. Eine Wippe 1105 mit beweglichem Stift 1130 ist ebenfalls gezeigt. Das innere Ende der Wippe 1105 greift in die Nut 1155 des in seiner Längsachse beweglichen Bolzens 1150. Durch Einrasten des Patronenverschlusses wird auf den Bolzen 1150 eine Kraft ausgeübt, so dass dieser sich in seiner Längsachse 1159 bewegt. Diese Bewegung wird über die Koppelung in der Nut 1155 auf die Wippe 1105 übertragen. Die Wippe ist dann in gekipptem Zustand und der bewegliche Stift 1130 übt eine Kraft auf das Gelenk-Implantat aus (nicht gezeigt) und fixiert dieses stabil am Instrumentenkopf 1100.

Da der Schaft 701 gemäss Fig. 7B ebenso wie der Instrumenten-Kopf 1100 denselben Patronenverschluss aufweisen, kann ein und derselbe Schaft 701 mit einem dazugehörigen Griff dazu verwendet werden, verschiedene Instrumenten-Köpfe aufzunehmen.

Fig. 12 zeigt einen Schnitt des alternativen Schafts 701 mit Patronenverschluss mit aufgesetztem Instrumenten-Kopf 1100. Der Instrumenten-Kopf und der Schaft sind mittels Patronenverschluss stabil verbunden.

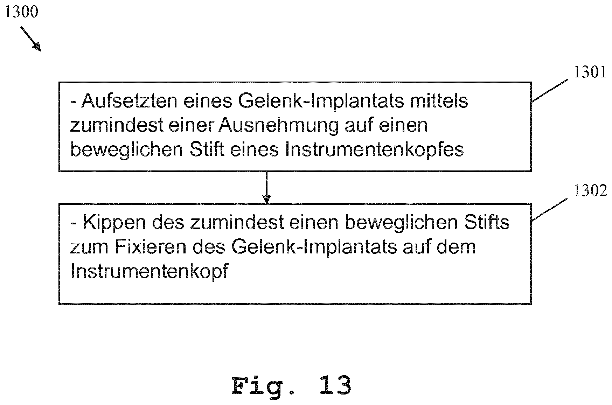

Fig. 13 zeigt ein Flussdiagramm zur Erläuterung des erfindungsgemäßen Verfahrens zur Fixierung eines Gelenk-Implantats.

In einem ersten Schritt 1301 wird ein Gelenk-Implantat 310, welches zumindest eine Ausnehmung 339 zum Aufnehmen eines Stifts aufweist, auf zumindest einen beweglichen Stift 330 eines Instrumenten-Kopfs 300A aufgesetzt. Bei der Durchführung dieses Schritts ist die Längsachse 331 des beweglichen Stifts parallel zur Längsachse der Ausnehmung zum Aufnehmen eines Stifts ausgerichtet, wodurch das Gelenk-Implantat 310 auf den beweglichen Stift 330 aufgesetzt werden kann, ohne dass es zum Verkanten des beweglichen Stifts 330 mit der Innenfläche der Ausnehmung kommt.

In einem zweiten Schritt 1302 wird das auf den Instrumenten-Kopf 300B aufgesetzte Gelenk-Implantat 310 auf dem Instrumenten-Kopf 300B fixiert, indem der zumindest eine bewegliche Stift 330 gekippt wird. Eine Kippbewegung des beweglichen Stifts 330 führt dazu, dass die Längsachse 332 des beweglichen Stifts nun nicht mehr parallel zu der Längsachse der Ausnehmung 339 zum Aufnehmen eines Stifts ist, wodurch es zu einer Kraftwirkung des gekippten Stifts 330 auf die Innenfläche der Ausnehmung 339 kommt. Aufgrund dieser Kraftwirkung wird das Gelenk-Implantat 310 auf dem Instrumenten-Kopf 330B fixiert.

Die Erfindung ist nicht auf das dargestellte Ausführungsbeispiel beschränkt.

## Patentansprüche

1. Ein Instrument zum Einsetzen eines Gelenk-Implantats (310), wobei das Instrument einen Instrumenten-Kopf (100, 300A, 300B), der mindestens einen beweglichen Stift (330) aufweist, und einen Schaft (301, 701A, 701B) hat,
wobei der bewegliche Stift (330) kippbar ist,
**dadurch gekennzeichnet,**
**dass** der Instrumenten-Kopf (300A, 300B) eine Auflagefläche (356) für das Gelenk-Implantat (310) aufweist, und
**dass** der Instrumenten-Kopf (200) in seiner Auflagefläche (256) mehrere feststehende Stifte (240) aufweist, deren Längsachsen (249) parallel zu der Längsachse (211, 711) des Schafts (301, 701A, 701B) sind.

2. Instrument nach Anspruch 1,
**dadurch gekennzeichnet,**
**dass** der Instrumenten-Kopf (300A, 300B)über den Schaft (301, 701A, 701B) des Instrumenten-Kopfs (300A, 300B) mit einem Griff (702) verbindbar ist, wobei die Längsachse des beweglichen Stifts (331) in einem ungekippten Zustand parallel zur Längsachse (311, 711) des Schafts (301, 701A, 701B) ist.

3. Instrument nach Anspruch 2,
**dadurch gekennzeichnet,**
**dass** der zumindest eine bewegliche Stift (330) derart kippbar ist, dass seine Längsachse (332) im gekippten Zustand nicht parallel zu der Längsachse (311, 711) des Schafts (701A, 701B) ist.

4. Instrument nach einem der Ansprüche 1 bis 3,
**dadurch gekennzeichnet,**
**dass** der zumindest eine bewegliche Stift (330) derart ausgeformt ist, dass er in zumindest eine Ausnehmung (339) des Gelenk-Implantats (310), welches auf der Auflagefläche (356) des Instrumenten-Kopfs (300A, 300B) aufgesetzt ist, eingreift und/oder, dass das Gelenk-Implantat (310) durch den gekippten Stift (330) auf der Auflagefläche (356) fixiert ist.

5. Instrument nach einem der Ansprüche 1 bis 4,
**dadurch gekennzeichnet,**
**dass** in den Instrumenten-Kopf (100) eine Ausnehmung (104) eingebracht ist, in welche eine bewegbare Wippe (105) eingesetzt ist.

6. Instrument nach Anspruch 5,
**dadurch gekennzeichnet,**
**dass** der zumindest eine bewegliche Stift (330) auf der Wippe (305) fixiert ist.

7. Instrument nach einem der Ansprüche 5 oder 6,
**dadurch gekennzeichnet,**
**dass** durch eine axiale Kraft entlang der Längsachse (311, 711) des Schafts (301, 701A, 701B) die Wippe (305) derart beweglich ist, dass ihre der axialen Kraft zugewandte Fläche (307) parallel zu einer Fläche (306) ist, welche die axiale Kraft ausübt.

8. Instrument nach Anspruch 7,
**dadurch gekennzeichnet,**
**dass** die Fläche der Wippe (307), auf welche die axiale Kraft wirkt, nicht parallel zu der Fläche (308) ist, auf welcher der zumindest eine bewegliche Stift (330) fixiert ist, und/oder
**dass** die Fläche (308), auf welcher der zumindest eine bewegliche Stift (330) fixiert ist, parallel zur Auflagefläche (356) des Instrumenten-Kopfs (300A) ist.

9. Instrument nach einem der Ansprüche 1 bis 4,
**dadurch gekennzeichnet,**
**dass** der Instrumenten-Kopf (100) zumindest einen entlang seiner Längsachse beweglichen Bolzen (1150) und mindestens eine bewegbare Wippe (1105) enthält.

10. Instrument nach Anspruch 9,
**dadurch gekennzeichnet,**
**dass** der zumindest eine bewegliche Stift (1130) auf der mindestens einen Wippe (1105) fixiert ist.

11. Instrument nach Anspruch 9 und 10,
**dadurch gekennzeichnet,**
**dass** der entlang der Längsachse bewegliche Bolzen (1150) und die mindestens eine bewegbare Wippe (1105) gekoppelt sind.

12. Instrument nach Anspruch 11,
**dadurch gekennzeichnet,**
**dass** im entlang der Längsachse beweglichen Bolzen (1150) mindestens eine umlaufende Nut (1155) angebracht ist.

13. Instrument nach Anspruch 12,
**dadurch gekennzeichnet,**
**dass** das dem zumindest einen beweglichen Stift (1130) entgegengesetzte Ende der Wippe (1105) in die mindestens eine umlaufende Nut (1155) des entlang der Längsachse beweglichen Bolzen (1150) eingreift.

14. Instrument nach Anspruch 7 oder 8 oder 11 oder 13,
**dadurch gekennzeichnet,**
**dass** die axiale Kraft erzeugbar ist, indem ein Gewinde (516, 716), das am Schaft (501, 701A, 701B) vorhanden ist und dessen Längsachse mit der Längsachse des Schafts (511, 711) zusammenfällt, in ein Gewinde (117) des Instrumenten-Kopfs (100) hineindrehbar ist.

15. Instrument nach Anspruch 7 oder 8 oder 11 oder 13,
**dadurch gekennzeichnet,**
**dass** die axiale Kraft erzeugbar ist, indem ein Patronenverschluss (726), der am Schaft (701A, 701B) vorhanden ist und dessen Längsachse mit der Längsachse (711) des Schafts zusammenfällt, mit einem entsprechenden Patronenverschluss-Gegenstück am Instrumenten-Kopf (100) gekoppelt wird.

16. Instrument nach einem der Ansprüche 1 bis 15,
**dadurch gekennzeichnet,**
**dass** in den Instrumenten-Kopf (200) eine Ausnehmung (218) eingebracht ist, in welche das dem Griff (702) abgewandte Ende (718) des Schafts (701A, 701B) einführbar ist.

17. Verfahren zum Fixieren eines Gelenk-Implantats (310) mittels eines Instruments ach einem der Ansprüche 1-16,
wobei das Gelenk-Implantat (310) auf eine Auflagefläche (356) des Instrumenten-Kopfs (300A, 300B) aufgesetzt wird,
und das Gelenk-Implantat (310) mittels zumindest einer Ausnehmung (339) auf den beweglichen Stift (330) und auf die mehreren feststehenden Stifte (340) des Instrumenten-Kopfs (300A) aufgesetzt wird, und
dass der bewegliche Stift (330) gekippt wird, um das Gelenk-Implantat (310) auf dem Instrumenten-Kopf (300B) zu fixieren.

## Claims

1. Instrument for inserting a joint implant (310), wherein the instrument has an instrument head (100, 300A, 300B), which comprises at least one movable post (330), and a shaft (301, 701A, 701B), wherein the movable post (330) is able to be tilted,
**characterized in that**
the instrument head (300A, 300B) has a bearing face (356) for the joint implant (310), and
**in that** the instrument head (200) in the bearing face (256) thereof has a plurality of stationary posts (240), the longitudinal axes (249) of the latter being parallel to the longitudinal axis (211, 711) of the shaft (301, 701A, 701B).

2. Instrument according to Claim 1,
**characterized in that**
the instrument head (300A, 300B) by way of the shaft (301, 701A, 701B) of the instrument head (300A, 300B) is connectable to a handle (702), wherein the longitudinal axis of the movable post (331) in a non-tilted state is parallel to the longitudinal axis (311, 711) of the shaft (301, 701A, 701B).

3. Instrument according to Claim 2,
**characterized in that**
the at least one movable post (330) is able to be tilted in such a manner that the longitudinal axis (332) thereof in the tilted state is not parallel to the longitudinal axis (311, 711) of the shaft (701A, 701B).

4. Instrument according to one of Claims 1 to 3,
**characterized in that**
the at least one movable post (330) is shaped in such a manner that said post (330) engages in at least one recess (339) of the joint implant (310), the latter being placed onto the bearing face (356) of the instrument head (300A, 300B), and/or **in that** the joint implant (310) is fixed on the bearing face (356) by the tilted post (330).

5. Instrument according to one of Claims 1 to 4,
**characterized in that**
a recess (104) into which a movable rocker (105) is inserted is incorporated in the instrument head (100).

6. Instrument according to Claim 5,
**characterized in that**
the at least one movable post (330) is fixed on the rocker (305) .

7. Instrument according to one of Claims 5 or 6,
**characterized in that**
the rocker (305), on account of an axial force along the longitudinal axis (311, 711) of the shaft (301, 701A, 701B), is movable in such a manner that the face (307) of said rocker (305) that faces the axial force is parallel to a face (306) which exerts the axial force.

8. Instrument according to Claim 7,
**characterized in that**
the face of the rocker (307) on which the axial force acts is not parallel to the face (308) on which the at least one movable post (330) is fixed, and/or
**in that** the face (308) on which the at least one movable post (330) is fixed is parallel to the bearing face (356) of the instrument head (300A).

9. Instrument according to one of Claims 1 to 4,
**characterized in that**
the instrument head (100) comprises at least one pin (1150), which is movable along the longitudinal axis thereof, and at least one movable rocker (1105).

10. Instrument according to Claim 9,
**characterized in that**
the at least one movable post (1130) is fixed on the at least one rocker (1105).

11. Instrument according to Claims 9 and 10,
**characterized in that**
the pin (1150), which is movable along the longitudinal axis, and the at least one movable rocker (1105) are coupled.

12. Instrument according to Claim 11,
**characterized in that**
at last one encircling groove (1155) is included in the pin (1150) which is movable along the longitudinal axis.

13. Instrument according to Claim 12,
**characterized in that**
the end of the rocker (1105) that is distal from the at least one movable post (1130) engages in the at least one encircling groove (1155) of the pin (1150) which is movable along the longitudinal axis.

14. Instrument according to Claim 7 or 8 or 11 or 13,
**characterized in that**
the axial force is able to be generated **in that** a thread (516, 716), the latter being present on the shaft (501, 701A, 701B) and the longitudinal axis thereof coinciding with the longitudinal axis of the shaft (511, 711), is able to be driven into a thread (117) of the instrument head (100).

15. Instrument according to Claim 7 or 8 or 11 or 13,
**characterized in that**
the axial force is able to be generated **in that** a stud lock (726), the latter being present on the shaft (701A, 701B) and the longitudinal axis thereof coinciding with the longitudinal axis (711) of the shaft, is coupled to a corresponding stud lock counterpart on the instrument head (100).

16. Instrument according to one of Claims 1 to 15,
**characterized in that**
a recess (218) into which the end (718) of the shaft (701A, 701B) that faces away from the handle (702) is able to be introduced is incorporated in the instrument head (200).

17. Method for fixing a joint implant (310) by means of an instrument according to one of Claims 1 to 16, wherein the joint implant (310) is placed onto a bearing face (356) of the instrument head (300A, 300B), and
the joint implant (310) by means of at least one recess (339) is placed onto the movable post (330) and onto the plurality of stationary posts (340) of the instrument head (300A), and
in that the movable post (330) is tilted so as to fix the joint implant (310) on the instrument head (300B).

## Revendications

1. Instrument pour insérer un implant articulaire (310), où l'instrument a une tête d'instrument (100, 300A, 300B), qui présente au moins une broche mobile (330), et une tige (301, 701A, 701B), où la broche mobile (330) est inclinable,
**caractérisé en ce que** la tête d'instrument (300A, 300B) présente une surface d'appui (356) pour l'implant articulaire (310), et
**en ce que** la tête d'instrument (200) présente dans sa surface d'appui (256) plusieurs broches fixes (240) dont les axes longitudinaux (249) sont parallèles à l'axe longitudinal (211, 711) de la tige (301 , 701A, 701B).

2. Instrument selon la revendication 1,
**caractérisé en ce que** la tête d'instrument (300A, 300B) peut être reliée à une poignée (702) par le biais de la tige (301, 701A, 701B) de la tête d'instrument (300A, 300B), où, dans un état non incliné, l'axe longitudinal de la broche mobile (331) est parallèle à l'axe longitudinal (311, 711) de la tige (301, 701A, 701B).

3. Instrument selon la revendication 2,
**caractérisé en ce que** la au moins une broche mobile (330) peut être inclinée de telle manière qu'à l'état incliné son axe longitudinal (332) n'est pas parallèle à l'axe longitudinal (311, 711) de la tige (701A, 701B).

4. Instrument selon l'une des revendications 1 à 3,
**caractérisé en ce que** la au moins une broche mobile (330) est conformée de telle manière qu'elle pénètre dans au moins un évidement (339) de l'implant articulaire (310), qui est placé sur la surface d'appui (356) de la tête d'instrument (300A, 300B) et/ou **en ce que** l'implant articulaire (310) est fixé par la broche inclinée (330) sur la surface d'appui (356).

5. Instrument selon l'une des revendications 1 à 4,
**caractérisé en ce qu'**un évidement (104) est ménagé dans la tête d'instrument (100), dans lequel une bascule pouvant être rendue mobile (105) est insérée.

6. Instrument selon la revendication 5,
**caractérisé en ce que** la au moins une broche mobile (330) est fixée sur la bascule (305).

7. Instrument selon l'une des revendications 5 ou 6,
**caractérisé en ce que**, par une force axiale le long de l'axe longitudinal (311, 711) de la tige (301, 701A, 701B), la bascule (305) est mobile de telle sorte que sa surface (307) faisant face à la force axiale est parallèle à une surface (306) qui exerce la force axiale.

8. Instrument selon la revendication 7,
**caractérisé en ce que** la surface de la bascule (307) sur laquelle agit la force axiale n'est pas parallèle à la surface (308) sur laquelle la au moins une broche mobile (330) est fixée, et/ou
**en ce que** la surface (308) sur laquelle la au moins une broche mobile (330) est fixée est parallèle à la surface d'appui (356) de la tête d'instrument (300A).

9. Instrument selon l'une des revendications 1 à 4,
**caractérisé en ce que** la tête d'instrument (100) contient au moins un tourillon (1150) mobile le long de son axe longitudinal et au moins une bascule pouvant être rendue mobile (1105).

10. Instrument selon la revendication 9,
**caractérisé en ce que** la au moins une broche mobile (1130) est fixée sur la au moins une bascule (1105).

11. Instrument selon la revendication 9 et 10,
**caractérisé en ce que** le tourillon (1150) mobile le long de l'axe longitudinal et la au moins une bascule pouvant être rendue mobile (1105) sont couplés.

12. Instrument selon la revendication 11,
**caractérisé en ce qu'**au moins une rainure circonférentielle (1155) est pratiquée dans le tourillon (1150) mobile le long de l'axe longitudinal.

13. Instrument selon la revendication 12,
**caractérisé en ce que** l'extrémité de la bascule (1105) opposée à la au moins une broche mobile (1130) pénètre dans la au moins une rainure circonférentielle (1155) du tourillon (1150) mobile le long de l'axe longitudinal.

14. Instrument selon la revendication 7 ou 8 ou 11 ou 13,
**caractérisé en ce que** la force axiale peut être produite par le fait qu'un filetage (516, 716), qui est présent sur la tige (501, 701A, 701B) et dont axe longitudinal coïncide avec l'axe longitudinal de la tige (511, 711), peut être vissé dans un filetage (117) de la tête d'instrument (100).

15. Instrument selon la revendication 7 ou 8 ou 11 ou 13,
**caractérisé en ce que** la force axiale peut être produite par le fait qu'une fermeture à mandrin (726), qui est présente sur la tige (701A, 701B) et dont l'axe longitudinal coïncide avec l'axe longitudinal (711) de la tige, est couplée avec un pendant de fermeture à mandrin correspondant sur la tête d'instrument (100).

16. Instrument selon l'une des revendications 1 à 15,
**caractérisé en ce qu'**un évidement (218) est ménagé dans la tête d'instrument (200), dans lequel l'extrémité (718) de la tige (701A, 701B) écartée de la poignée (702) peut être insérée.

17. Procédé de fixation d'un implant articulaire (310) au moyen d'un instrument selon l'une des revendications 1 à 16,
où l'implant articulaire (310) est placé sur une surface d'appui (356) de la tête d'instrument (300A, 300B), et l'implant articulaire (310) est placé sur la broche mobile (330) et sur les plusieurs broches fixes (340) de la tête d'instrument (300A) au moyen d'au moins un évidement (339), et en ce que la broche mobile (330) est inclinée pour fixer l'implant articulaire (310) sur la tête d'instrument (300B).
